Europäisches Patentamt

(19) European Patent Office

Office européen des brevets

(11) EP 0 842 687 B1

## (12) FASCICULE DE BREVET EUROPEEN

(45) Date de publication et mention de la délivrance du brevet:
24.09.2003 Bulletin 2003/39

(51) Int Cl.⁷: $B01D\ 15/02$, $B01J\ 8/04$

(21) Numéro de dépôt: 97402698.1

(22) Date de dépôt: 12.11.1997

(54) **Dispositif d'équilibrage de pression et de rinçage dans une enceinte**

Druckausgleichs- und Spülvorrichtung in einem Behälter

Pressure equalising and rinsing device in a container

(84) Etats contractants désignés:
**DE ES GB IT NL**

(30) Priorité: **19.11.1996 FR 9614186**

(43) Date de publication de la demande:
**20.05.1998 Bulletin 1998/21**

(73) Titulaire: **Institut Français du Pétrole
92506 Rueil Malmaison Cédex (FR)**

(72) Inventeurs:
- **Renard, Pierre
78860 Saint Nom La Breteche (FR)**
- **Dessapt, Jean Paul
78650 Beynes (FR)**
- **Callebert, Olivier
92500 Rueil Malmaison (FR)**

(56) Documents cités:
**US-A- 3 946 104**       **US-A- 5 415 773**

## Description

L'invention concerne un réacteur comportant au moins une cloison interne, généralement métallique délimitant deux compartiments dans lesquels circulent deux fluides différents ou non et dans lesquels des variations de pression de part et d'autre de la cloison peuvent se produire.

L'invention concerne aussi un procédé de rinçage de fonds de réacteur isolés par une cloison et subissant une variation minime de pression, généralement par à-coups.

Elle s'applique tout particulièrement au dispositif mettant en oeuvre un lit mobile simulé, par exemple un dispositif d'adsorption chromatographique d'un mélange de xylènes pour séparer du paraxylène ou d'un mélange d'hydrocarbures aliphatiques pour séparer les n-alcanes des iso-alcanes, ou les n-alcènes des iso-alcènes. C'est ce type de dispositif d'adsorption qui sera décrit pour exemple dans cette demande de brevet.

Dans un réacteur sous pression, très rapidement au-delà d'un certain diamètre (supérieur à 1 m par exemple) les fonds sont généralement de forme elliptique ou hémisphérique de façon à mieux supporter mécaniquement la pression.

Dans certains cas la partie réactionnelle elle-même n'épouse pas, ou ne peut épouser directement la forme de ces fonds.

C'est le cas par exemple de réacteurs mettant en oeuvre de la chromatographie en phase liquide ou vapeur pour laquelle la surface inférieure et supérieure des lits d'adsorbant doit être parfaitement plane pour obtenir un front d'avancement des fluides le plus plan possible.

Pour les besoins du procédé, on est donc amené dans ce type de cas à isoler la partie réactionnelle du volume constitué par le ou les fonds, au moyen d'une cloison interne de forme appropriée, cloison plane munie d'une grille dans le cas de la chromatographie liquide ou vapeur :

- la cloison plane isole le fluide procédé (fluide process) du fluide contenu dans le ou les fonds.
- Une grille placée près de la cloison et de forme sensiblement parallèle à la cloison plane assure le maintien de l'adsorbant dans le volume qui lui est réservé, tout en permettant la circulation du fluide à travers le lit et sa collecte vers l'extérieur, ou son injection depuis l'extérieur du réacteur.

Cette cloison interne n'a pas une forme appropriée pour supporter la pression et on est donc amené à devoir équilibrer les pressions de part et d'autre de cette cloison, à savoir la pression côté procédé et la pression côté fonds.

La façon la plus simple d'équilibrer ces pressions est de pratiquer une ouverture à travers cette cloison : la moindre différence de pression se traduit aussitôt par un transfert de fluide et le système s'équilibre rapidement notamment si le fluide est un liquide.

Cependant, dans certains procédés, tels que la chromatographie en lit mobile simulé, le fluide qui circule côté procédé pendant un cycle est tantôt pur et tantôt impur, et par ailleurs il se produit de légers à-coups de pression inhérents au procédé, dûs aux ouvertures et fermeture de vannes et aux changements de débit, et donc des transferts de matière à travers l'ouverture de la cloison au cours de ce même cycle.

Il s'ensuit donc que le volume de fluide contenu dans un fond se pollue progressivement, chaque à-coup de pression induisant un transfert de fluide pur ou impur à travers l'ouverture pratiquée dans la cloison, en provenance de la zone d'adsorption.

Inversement, le volume pollué contenu dans un fond peut à son tour polluer le fluide procédé lorsque celui-ci se trouve être pur à un moment du cycle et lorsqu'il y a un à-coup de pression.

Pour éviter ce phénomène, une solution est de prévoir l'injection d'un fluide de rinçage propre dans le fond concerné.

Cette solution envisageable pour un petit diamètre est inapplicable pour de plus gros diamètres : le débit de rinçage étant très petit devant le volume du fond, si celui-ci se pollue accidentellement, la pollution se répand par mélange dans tout le volume de fond et le fluide de rinçage ne peut assurer rapidement le remplacement du volume pollué par du fluide de rinçage propre, à moins d'utiliser un très fort débit de rinçage, incompatible avec le procédé, car ce débit s'ajoute au fluide procédé.

Pour améliorer ce dispositif d'équilibrage en induisant le minimum de pollution, le brevet US-5,415,773 décrit l'arrangement suivant :

- L'ouverture dans la cloison est prolongée par un conduit dit de confinement, de volume minimum calculé pour que l'interface entre le fluide procédé côté adsorbant, et le fluide côté fond, reste contenu dans ce volume lors d'à-coups de pression.

Selon ce brevet, la pollution est normalement limitée à ce conduit.

- De plus ce volume de confinement dans ce conduit est connecté à une chambre de rinçage totalement ouverte à une extrémité dans le volume de fond et connectée à l'autre extrémité à une ligne de purge vers l'extérieur.

- Un fluide extérieur de rinçage est injecté en continu dans la chambre d'équilibrage à un débit égal ou très légèrement supérieur au débit de purge.

- De cette façon :

  - Le débit de fluide de rinçage envoyé dans le fluide procédé à travers l'ouverture de la cloison est nul ou réduit au minimum.

- Toute pollution accidentelle de volume supérieur à celui du conduit de confinement, (l'interface fluide procédé/fluide de rinçage se déplaçant accidentellement au-delà du conduit) se trouve entraînée et rincée dans la chambre de rinçage et évacuée à l'extérieur par la ligne de purge.

Ce dispositif présente les inconvénients suivants :

- Bien que l'injection du fluide de rinçage soit réalisée par un fluide propre, on ne peut empêcher le volume du fond (chambre d'équilibrage) de se polluer en partie par des fuites existantes issues du fluide procédé,

  - au niveau de la cloison interne composée de plusieurs éléments assemblés et disposés côte à côte et jamais parfaitement étanche,

  - au niveau du conduit (ou manifold) permettant la circulation du fluide procédé à travers le fond depuis l'extérieur vers le lit d'adsorbant (ou vice-versa). Ce conduit peut être composé d'éléments assemblés par des brides pouvant être source de fuites.

  Toutes ces pollutions peuvent être en partie transférées via ce conduit de confinement, l'orifice et les défauts de non-étanchéité de la cloison, dans le fluide procédé, ce qui est préjudiciable en terme de pureté.

- Le volume du conduit de confinement étant très faible, il ne peut assurer dans tous les cas le confinement de la pollution à l'intérieur de ce volume. L'interface fluide procédé/fluide de rinçage pourra ainsi dans certains cas se déplacer d'une façon telle, notamment dans le cas de diamètre de réacteur important, qu'il se situera tantôt nettement dans la chambre de rinçage, tantôt nettement dans la zone d'adsorption, ce qui entraîne automatiquement une pollution réciproque du produit côté procédé et côté chambre de rinçage.

- La recherche d'un débit nul ou minimum de la fuite de rinçage à travers l'ouverture de la cloison signifie que le débit d'injection de rinçage est très voisin ou égal au débit de purge, et qu'il n'y a pas de différence significative de pression (pas de surpression contrôlée) entre le fond (chambre d'équilibrage) et la partie procédé. Cette différence de pression n'étant pas contrôlée, on a donc toujours un risque de pollution depuis la chambre d'équilibrage vers la partie procédé (ou inversement) à travers toutes les ouvertures existantes : ouverture pratiquée dans la cloison, ouvertures dues à l'assemblage imparfait des pièces constituant la cloison ou le manifold.

L'arrière plan technologique est aussi illustré par le brevet US-3,946,104.

Un objet de l'invention est de remédier aux inconvénients mentionnés ci-devant.

Un autre objet de l'invention est de proposer un réacteur sous pression comportant des cloisons ou des plaques de distribution planes, qui éliminent sensiblement les problèmes associés à un rinçage en tête et/ ou en fond de réacteur.

Un autre objet de l'invention est d'améliorer les performances d'un lit mobile, simulé, notamment mis en oeuvre dans des réacteurs de très grand diamètre.

Plus précisément, l'invention concerne un dispositif d'équilibrage de pression et de rinçage dans une enceinte ayant au moins une extrémité de forme concave, l'enceinte comprenant une chambre de séparation chromatographique ou chambre réactionnelle, au moins un plateau de distribution ou de collecte située dans l'enceinte, une chambre d'équilibrage de pressions définie au moins en partie par l'extrémité de ladite enceinte et par une première face du plateau, la chambre de séparation ou réactionnelle étant définie au moins en partie par une seconde face dudit plateau de distribution ou de collecte, un premier conduit faisant communiquer un premier fluide avec le plateau de distribution ou de collecte, la chambre de séparation ou réactionnelle communiquant avec la chambre d'équilibrage par une chambre de rinçage (flush chamber) comprenant un premier orifice, le dispositif étant caractérisé en ce qu'une ligne d'alimentation en un second fluide sensiblement pur ou sensiblement non contaminé est connecté directement à la chambre de rinçage en un point tel qu'une partie dudit second fluide puisse circuler vers la chambre d'équilibrage de pressions via un autre orifice et que la partie restante puisse circuler vers la chambre de séparation ou réactionnelle via le premier orifice, et en ce que ladite chambre d'équilibrage de pressions est reliée à un moyen d'évacuation de fluide qu'elle contient.

Le second fluide introduit latéralement dans la chambre de rinçage est généralement un solvant de désorption du produit recherché, le produit recherché lui-même, par exemple le paraxylène, ou leur mélange, du toluène et du paraxylène par exemple.

La combinaison d'une chambre de rinçage et d'un plateau de distribution selon l'invention présente les avantages suivants: en introduisant directement dans la chambre de rinçage un fluide non contaminé, du solvant par exemple, on ne fait pas pénétrer, dans la chambre d'adsorption, du fluide provenant de la chambre d'égalisation de pressions, susceptible d'être pollué par des produits provenant de fuites de toutes sortes au niveau de la cloison, du plateau de distribution ou du circuit de distribution des hydrocarbures entrant dans la section de colonne chromatographique adjacente au plateau de distribution. Toute contamination s'avérerait préjudiciable en terme de pureté.

Selon une caractéristique de l'invention, la chambre de rinçage est sensiblement tubulaire.

Selon une autre caractéristique, la ligne d'alimentation du second fluide peut être connectée à la chambre de rinçage en un point correspondant à un volume à l'intérieur de ladite chambre ou dudit tube représentant 30 à 90 % du volume total de la chambre de rinçage, ce volume étant déterminé à partir de la face du plateau de distribution. On a observé de meilleurs résultats lorsque le point de connexion de la ligne d'alimentation sur la chambre de rinçage correspondait à un volume représentant 50 à 80 % du volume total de la chambre. Dans ces conditions, lors des à-coups de pression, le volume de la zone contaminée par la remontée du fluide principal dans la chambre de rinçage restera toujours inférieur à ce volume de garde et donc ne contaminera pas la chambre d'équilibrage de pression. En d'autres termes il y aura une circulation d'une partie du second fluide vers la chambre d'adsorption mais lorsqu'une surpression dans la chambre d'équilibrage se manifeste sur le plateau distributeur, du fluide principal provenant de la chambre d'adsorption s'introduit transitoirement dans le volume de garde de la chambre de rinçage sans le dépasser puis est renvoyé lors du retour à l'équilibre avec le second fluide dans la chambre d'adsorption. En moyenne, la circulation d'une partie du second fluide s'effectuera toujours dans la même direction (vers la chambre d'adsorption) et il n'y aura donc pas de transfert du fluide principal de la chambre d'adsorption vers la chambre d'équilibrage de pression.

La chambre de rinçage et plus particulièrement le volume de garde ou de confinement de la pollution sera calculé en fonction des fluctuations maximales de pression, c'est-à-dire de la déformation de la cloison du plateau de distribution ou de collecte, consécutive à ces fluctuations de pression.

Ce volume peut être au plus égal à :

$$V \ (cm^3) = S(m^2) \times 0,01 \ (m) \ ,$$

S étant la section du réacteur, et de préférence au plus égal à S x 0,001, ce qui permet de couvrir les plus importants transferts de fluides à travers l'orifice de la cloison induits par les à-coups de pression du procédé, dans le cas des réacteurs de très grand diamètre.

Le débit de rinçage est, en règle générale, significativement sensiblement supérieur au débit de purge de façon à assurer en permanence un débit significativement positif de fluide propre vers le côté procédé à travers l'orifice de la cloison.

On obtient ainsi :

- Un rinçage systématique du volume de garde ou de confinement par un fluide propre.

- Une légère surpression contrôlée de la chambre d'équilibrage par rapport au côté procédé. Il s'ensuit que si fuites il y a à travers les parties assemblées de la cloison ou du manifold, elles seront toujours composées de fluide propre ne pouvant polluer le procédé.

De cette façon, les fuites éventuelles de fluide depuis la chambre de rinçage vers le côté procédé à travers les assemblages imparfaits de la cloison seront de préférence composés de fluide propre.

Selon une caractéristique avantageuse du dispositif, la chambre de rinçage, de préférence tubulaire, peut comporter un orifice de sortie, côté chambre d'égalisation de pression, au voisinage immédiat de la face du plateau, ce qui permet de mieux balayer par un fluide propre les produits indésirables qui proviennent d'éventuelles fuites au voisinage du plateau de distribution ou du collecteur et qui sont concentrés à ce niveau et de garder propre le fluide au voisinage de la cloison. Ce drainage est d'autant mieux réalisé que le moyen d'évacuation du fluide contenu dans la chambre d'équilibrage de pressions est situé aussi au voisinage immédiat du plateau et de préférence dans une direction sensiblement diamétralement opposée à celle prise par le fluide sortant de l'orifice de sortie de la chambre de rinçage.

Avec une forme préférée en U par exemple, la fonction de drainage dans la partie inférieure de la chambre d'équilibrage de pression sera bien réalisée.

Ce dispositif de rinçage et d'équilibrage de pressions est particulièrement utile dans des procédés chromatographiques de séparation, par exemple par adsorption sur un adsorbant, dans les procédés catalytiques et dans les procédés d'adsorption réactives, mettant en oeuvre un solide particulaire. Il peut être opéré en présence d'un fluide gazeux, liquide, mixte, supercritique ou subcritique.

Il peut s'appliquer également à des procédés n'impliquant pas la mise en oeuvre de solides particulaires.

Il s'applique particulièrement en phase liquide, notamment dans le procédé d'adsorption en lit mobile simulé tels que décrits dans les brevets US 2 985 589, US 4 498 991, EP-A-679 421, EP-A-688 590, EP-A-688 589 et US 5 284 992, au cours duquel se manifestent de brusques variations de pression dues à la succession d'ouvertures et de fermetures de vannes.

L'invention concerne aussi un procédé d'équilibrage de pression et de rinçage dans un dispositif mettant en oeuvre un procédé de séparation chromatographique par exemple par adsorption, d'un mélange de composés sur un solide particulaire (adsorbant) de façon à séparer au moins un de ces composés. Plus précisément, ce procédé concerne la tête d'un réacteur et/ou le fond d'un réacteur qui est séparé de la zone de séparation chromatographique ou de la zone de réaction par un plateau de distribution ou de collecte, suivant le cas.

De manière plus détaillée, l'invention est relative à un procédé d'équilibrage de pression et de rinçage dans une chambre de séparation chromatographique ou dans une chambre réactionnelle comportant à une extrémité une zone d'équilibrage de pression, et une zone chromatographique ou réactionnelle dans laquelle on fait circuler un premier fluide (fluide process) via un plateau de distribution ou de collecte séparant la zone d'équilibrage de pression et la zone chromatographique ou réactionnelle , le procédé étant caractérisé en ce qu'on fait circuler un second fluide choisi dans le groupe formé par un solvant, un produit recherché et leur mélange dans une zone de rinçage faisant communiquer ladite zone chromatographique ou réactionnelle et la zone d'équilibrage de pression, en introduisant ledit second fluide dans la zone de rinçage par une liaison directe latérale, de telle sorte qu'une partie du second fluide circule de ladite chambre de rinçage vers la chambre d'équilibrage de pressions et que la partie restante du second fluide circule de la chambre de rinçage vers la chambre chromatographique ou réactionnelle et en ce que l'on fait évacuer une quantité d'un fluide provenant de la chambre d'équilibrage de pressions correspondant à la quantité de fluide sortant de la zone de rinçage.

Le solide particulaire peut être un adsorbant dans le cas d'une zone chromatographique ou un catalyseur dans le cas d'une zone réactionnelle. Dans certains cas, la zone réactionnelle peut ne pas contenir de solides particulaires.

Selon une caractéristique du procédé, on peut introduire le second fluide dans la chambre de rinçage à un débit $d_1$ au plus égal à 0,2 % du débit correspondant au premier fluide distribué dans la première section de colonne en contact avec le plateau de distribution. Avantageusement, ce débit $d_1$ peut être au plus égal à 0,1 % du débit du premier fluide et en particulier compris entre 0,02 et 0,05 %.

Selon une autre caractéristique du procédé, on fixe le débit $d_1$ du second fluide pénétrant directement dans la chambre de rinçage, on contrôle un débit $d_2$ de fluide quittant la chambre d'équilibrage de pression de telle façon que le débit $d_3$ de second fluide s'écoulant dans la chambre d'adsorption via l'orifice de sortie de la chambre de rinçage, soit au moins égal à 5 % du débit $d_1$ du second fluide introduit dans la chambre de rinçage. Avantageusement, ce débit $d_3$ est au moins égal à 10 % et de préférence compris entre 40 % et 60 % du débit $d_1$.

L'invention sera mieux comprise au vu des figures suivantes illustrant de manière schématique un mode de réalisation du dispositif et un exemple de mise en oeuvre du procédé, parmi lesquelles :

- la figure 1 montre en coupe axiale une enceinte d'adsorption en lit mobile simulé dont la tête et le fond contiennent respectivement une chambre d'équilibrage de pressions combinée à une chambre de rinçage communiquant avec la chambre d'adsorption ;
- la figure 2 illustre de manière plus précise en coupe longitudinale une tête de l'enceinte de séparation comportant le dispositif selon l'invention.

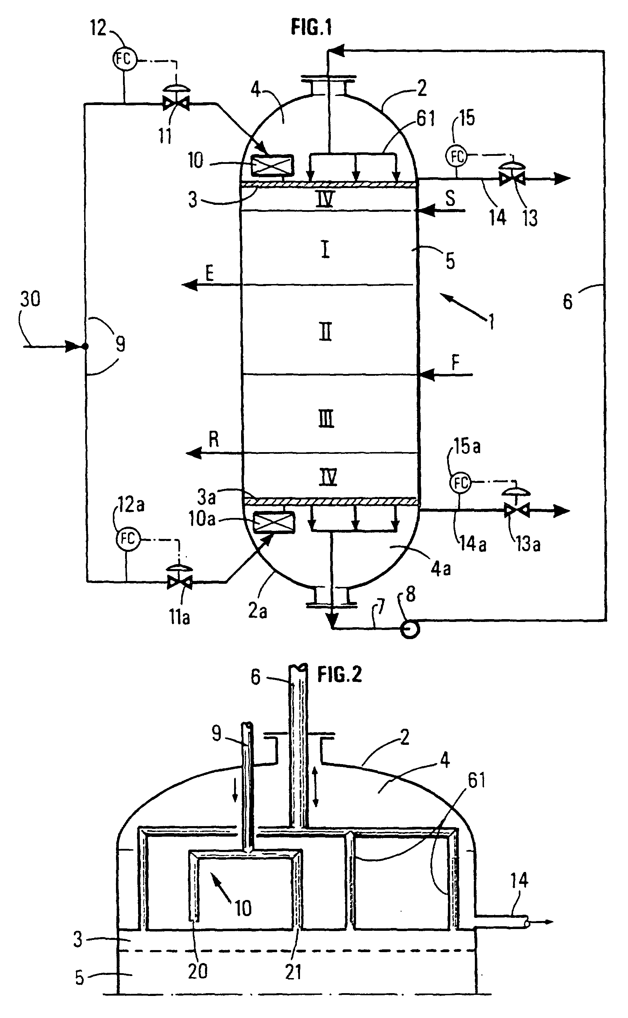

Selon la figure 1, une enceinte cylindrique 1 de forme allongée ayant à une extrémité un fond hémisphérique 2 comporte un plateau de distribution 3 d'un fluide principal alimenté par une ligne 6 et une pluralité de lignes 61 de distribution (voir figure 2). Ce plateau de distribution sépare une chambre d'équilibrage de pression 4 d'une chambre d'adsorption 5 proprement dit en dessous du plateau et alimente ladite chambre d'adsorption. La chambre d'adsorption 5 fonctionnant en lit mobile à contre-courant simulé comprend une pluralité de sections de colonnes, 24 par exemple, remplies d'un adsorbant zéolithique tel qu'une zéolithe X ou Y échangée au baryum et définissant quatre zones :

- une zone I de désorption entre une alimentation en solvant S et un soutirage d'extrait E,
- une zone II de purification entre une alimentation en charge F et un soutirage d'extrait E,
- une zone III d'adsorption comprise entre un soutirage de raffinat et l'alimentation en charge F,
- une zone IV tampon comprise entre l'alimentation en solvant S et le soutirage de raffinat R.

Sur la figure 1, le fluide sortant d'une section de colonne de la zone IV via un plateau de collecte, des lignes de collecte non représentées et une ligne 7 de recyclage est renvoyé par une pompe 8 et la ligne 6 d'alimentation en tête de la chambre d'adsorption pour être redistribué sur le plateau de distribution 3 dans une autre section de colonne de la zone IV.

Dans le fond hémisphérique contenant la chambre d'équilibrage de pressions 4, une chambre de rinçage 10 en forme de U est alimentée grâce à une ligne directe 9 par un désorbant tel que le toluène, provenant d'une alimentation 30. Une ligne d'évacuation 14 soutire un fluide contenu dans la chambre d'équilibrage de pressions. Des moyens de contrôle de débit 12 et 15 comprenant des vannes de régulation 11 et 13 respectivement sur les lignes 9 et 14 contrôlent le débit de désorbant introduit directement dans la chambre de rinçage et celui du fluide soutiré directement de la chambre d'équilibrage de pressions.

De manière plus détaillée, selon la figure 2, le fond supérieur 2 de forme hémisphérique ou elliptique, ferme l'extrémité supérieure de l'enceinte 1. La première face du plateau de distribution 3 définit côté extrémité la chambre d'équilibrage de pressions 4 tandis que la deuxième face de ce plateau définit la limite supérieure de la chambre d'adsorption proprement dit 5 qui contient l'adsorbant. La ligne 6 alimente par une tuyauterie ramifiée (manifold) 61 le plateau de distribution 3 en un fluide principal (fluide process) qui est celui qui provient de la dernière zone en contact avec le plateau de distribution inférieur, en l'occurrence la zone IV. Ce plateau de distribution permet à son tour d'alimenter en ce fluide principal l'adsorbant de la chambre 5.

La chambre de rinçage 10 contenue dans la zone d'équilibrage de pressions 4 reçoit directement du désorbant de débit $d_1$ en provenance de la ligne 9, par une liaison directe, de préférence perpendiculaire, judicieusement située entre un premier orifice de sortie 21 de la chambre de rinçage en contact avec le plateau de distribution et un deuxième orifice de sortie 20. Cet orifice de sortie 20 est avantageusement situé au voisinage immédiat du plateau de distribution et à proximité de sa périphérie. Une partie du désorbant de débit $d_2$ provenant de la ligne 9 pénètre dans la chambre d'équilibrage de pressions par l'orifice 20. Elle se mélange en partie avec le fluide de la chambre d'équilibrage de pressions qui contient habituellement du désorbant et éventuellement des impuretés dues à de multiples petites fuites provenant du plateau distributeur et du circuit de distribution 61 du fluide principal dont la composition change périodiquement au rythme de la progression des alimentations et des soutirages sur les sections de colonnes.

La partie restante de désorbant de débit $d_3$ de la ligne 9 s'écoule de la chambre de rinçage vers le plateau 3 de distribution et vers l'adsorbant du réacteur 5, à travers l'orifice de sortie 21 en contact avec ledit plateau. Elle représente typiquement en moyenne 50 % du débit de désorbant de la ligne 9.

Le débit $d_2$ de désorbant qui traverse l'orifice de sortie 20 est évacué avec les impuretés contaminantes par la canalisation de sortie 14 située sur la paroi extérieure de la chambre d'équilibrage 4. Ce soutirage de fluide de débit $d_2$ s'avère d'autant plus efficace que la canalisation est positionnée à proximité du plateau de distribution et de préférence en un point sensiblement diamétralement opposé à l'entrée de fluide de débit $d_2$ pénétrant dans la chambre 4. Le flux de fluide opère un effet de lavage qui draine en effet les contaminations essentiellement concentrées au voisinage du plateau de distribution.

Le dispositif d'équilibrage de pression et de rinçage fonctionne de la manière suivante. On fixe un débit $d_1$ de désorbant généralement au plus égal à 0,1 % du débit du fluide principal entrant dans la chambre d'adsorption 5, grâce à la vanne de contrôle 11 et du régulateur de débit 12. On régule un débit $d_2$ de sortie de fluide de la chambre d'équilibrage de pression par la vanne de régulation 13 et le régulateur 15, dans des conditions telles qu'en moyenne un débit $d_3$ de désorbant de composition identique à celle du désorbant amené par la ligne 9 s'écoule de la chambre de rinçage vers l'adsorbant via l'orifice 21.

Cette disposition et ce mode d'opération permettent par ailleurs de contrôler les pressions de part et d'autre du plateau de distribution. En effet, lorsqu'une dépression se manifeste dans la chambre d'adsorption, la membrane (ou cloison) du plateau distributeur 3 se déforme, ce qui fait remonter transitoirement du fluide procédé dans la chambre de rinçage, au plus jusqu'au point de jonction de la ligne 9 sur cette chambre. Le volume ainsi occupé par le fluide procédé représente 50 à 80 % du volume total de la chambre de rinçage. Le retour presque instantané à l'équilibre se traduit par le retour du fluide procédé emmagasiné dans ce volume de garde dans la chambre d'adsorption de sorte qu'aucune trace de fluide procédé n'est évacuée par l'orifice de sortie 20 dans la chambre d'équilibrage 4.

On a décrit un fond supérieur de réacteur, mais comme le montre la figure 1, on peut rencontrer les mêmes moyens avec les mêmes références indicées (avec un a tel que 2a, 3a, 4a) dans le fond inférieur du réacteur. Le plateau de distribution devient un plateau de collecte de fluide principal associé à un manifold de collecte et le fluide principal quittant la dernière zone est recyclé vers le fond de tête du réacteur grâce à la ligne de recyclage 7 et la pompe 8. Un débit $d_1$ de désorbant ou autre produit pur égal ou différent de celui introduit en tête est introduit directement et latéralement dans la chambre de rinçage 10a. Ce débit $d_1$ se divise en un débit $d_2$ circulant dans la chambre 4a d'équilibrage via la chambre de rinçage 10a et en un débit $d_3$ traversant l'orifice de sortie sur le plateau 3a de collecte, en communication avec la chambre d'adsorption 5. Le fluide contenu dans la chambre d'équilibrage des pressions est soutiré sous un débit $d_2$ par la canalisation d'évacuation 14a.

**Revendications**

1. Enceinte munie d'un dispositif d'équilibrage de pression et de rinçage, ladite enceinte (1) ayant au moins une extrémité de forme concave, et comprenant

   - une chambre (5) de séparation chromatographique ou une chambre réactionnelle,
   - au moins un plateau de distribution (3) ou de collecte (3a) située dans l'enceinte et communiquant avec ladite chambre (5) de séparation ou réactionnelle,
   - une chambre (4, 4a) d'équilibrage de pressions,
     ladite chambre (4, 4a) d'équilibrage étant définie au moins en partie par l'extrémité de ladite enceinte et par une première face du plateau, et ladite chambré (5) de séparation ou réactionnelle étant définie au moins en partie par une seconde face dudit plateau de distribution ou de collecte,
   - un premier conduit (6) faisant communiquer un premier fluide avec le plateau de distribution ou de collecte,
   - une chambre de rinçage (10),

la chambre de rinçage (10) communiquant avec la chambre d'équilibrage (4), au moyen d'un premier orifice (20) et avec le plateau de distribution (3) ou de collecte (3a) au moyen d'un second orifice (21), le dispositif étant **caractérisé en ce qu'**une ligne (9) d'alimentation en un second fluide sensiblement pur ou sensiblement non contaminé est connecté directement à la chambre de rinçage en un point tel qu'une partie dudit second fluide puisse circuler de la chambre de rinçage (10) vers la chambre d'équilibrage de pressions via le premier orifice de sortie (20) et que la partie restante puisse circuler de la chambre de rinçage (10) vers la chambre de séparation ou réactionnelle via le second orifice (21), et **en ce que** ladite chambre d'équilibrage de pressions (4, 4a) est reliée à un moyen (14) d'évacuation de fluide qu'elle contient.

2. Enceinte selon la revendication 1 dans lequel la chambre de rinçage est tubulaire.

3. Enceinte selon la revendication 1 ou 2 dans lequel la chambre de rinçage est située à l'intérieur de la chambre d'équilibrage (4, 4a) et délimitée par le plateau de distribution (3) ou de collecte (3a) et dans lequel la ligne d'alimentation (9) du second fluide est connectée à la chambre de rinçage (10) en un point correspondant à un volume de la chambre de rinçage représentant 30 à 90 % du volume total de ladite chambre, déterminé à partir de la première face du plateau.

4. Enceinte selon la revendication 3 dans lequel ledit point de connexion correspond à un volume représentant 50 à 80 % du volume total de la chambre de rinçage.

5. Enceinte selon l'une des revendications 1 à 4 dans lequel la chambre de rinçage comporte l'orifice (20) de sortie, côté chambre d'équilibrage de pression, au voisinage immédiat de la première face du plateau.

6. Enceinte selon l'une des revendications 2 à 5 dans lequel le moyen d'évacuation (14) du fluide de la chambre d'équilibrage de pression est situé au voisinage immédiat de ladite première face du plateau.

7. Enceinte selon l'une des revendications 5 à 6, dans lequel l'orifice de sortie (20) de la chambre de' rinçage, côté chambre d'équilibrage de pressions, est sensiblement diamétralement opposé au moyen d'évacuation (14) de fluide contenu dans la chambre d'équilibrage de pressions.

8. Enceinte selon l'une des revendications 1 à 7 dans lequel la chambre de séparation chromatographique est un lit mobile simulé.

9. Procédé d'équilibrage de pression et de rinçage dans une chambre de séparation chromatographique ou dans une réactionnelle comportant à une extrémité une zone (4) d'équilibrage de pression, et une zone chromatographique ou réactionnelle (5) dans laquelle on fait circuler un premier fluide via un plateau de distribution (3) ou de collecte (3a) séparant la zone d'équilibrage de pression et la zone chromatographique ou réactionnelle , le procédé étant **caractérisé en ce qu'**on fait circuler un. second fluide, choisi dans le groupe formé par un solvant, un produit recherché et leur mélange dans une zone de rinçage (10) faisant communiquer ladite zone chromatographique ou réactionnelle et la zone d'équilibrage de pression, en introduisant ledit second fluide dans la zone de rinçage par une liaison directe, de telle sorte qu'une partie du second fluide circule de ladite chambre de rinçage vers la zone (4) d'équilibrage de pressions et que la partie restante du second fluide circule de la chambre de rinçage vers la zone (5) chromatographique ou réactionnelle et **en ce que** l'on fait évacuer une quantité d'un fluide provenant de la chambre d'équilibrage de pressions correspondant à la quantité de fluide sortant de la zone de rinçage.

10. Procédé selon la revendication 9 dans lequel la liaison directe sur la zone de rinçage est positionnée de telle façon qu'on détermine un volume de garde entre le point de liaison et le plateau de distribution ou de collecte, représentant 30 à 90 % du volume de la zone de rinçage et suffisamment grand pour qu'il n'y ait pas de transfert de fluide de ta zone chromatographique ou réactionnelle vers la zone d'équilibrage de pressions lors des à-coups de pression.

11. Procédé selon la revendication 9 ou 10 dans lequel on introduit le second fluide à un débit $d_1$ au plus égal à 0,2 % du débit correspondant au premier fluide.

12. Procédé selon l'une des revendications 9 à 11 dans lequel on fixe un débit $d_1$ du second fluide pénétrant directement dans la zone de rinçage, on contrôle un débit $d_2$ de fluide quittant la zone d'équilibrage de pressions de telle façon que le débit $d_3$ de second fluide via l'orifice (21) s'écoulant dans la zone (5) chromatographique ou réactionnelle soit au moins égal à 5% du débit $d_1$, avantageusement au moins égal à 10 %, et de préférence compris entre 40 et 60 % du débit $d_1$.

13. Utilisation de l'enceinte selon l'une des revendications 1 à 8 pour la séparation chromatographique d'un mélange d'hydrocarbures comportant des xylènes, de préférence en lit mobile simulé.

**14.** Utilisation de l'enceinte selon l'une des revendications 1 à 8 pour la séparation chromatographique d'un mélange d'hydrocarbures aliphatiques.

**Patentansprüche**

**1.** Behälter ausgestattet mit einer Druckausgleichs- und Spülvorrichtung, wobei der besagte Behälter (1) mindestens ein Ende von konkaver Form hat und umfassend

a. eine chromatographische Separationskammer (5) oder eine Reaktionskammer,

b. mindestens eine in dem Behälter gelegene und mit der besagten Separationskammer (5) oder Reaktionskammer in Verbindung stehende Verteilebene (3) oder Sammelebene (3a),

c. eine Druckausgleichskammer (4, 4a), wobei die besagte Druckausgleichskammer (4, 4a) zumindest teilweise durch das Ende des besagten Behälters und durch eine erste Stirnfläche der Ebene begrenzt ist und wobei die besagte Separätionskammer (5) oder Reaktionskammer zumindest teilweise durch eine zweite Stirnfläche der besagten Verteil- oder Sammelebene begrenzt ist,

d. eine erste Leitung (6), die ein erstes Fluid mit der Verteil- oder Sarnmelebene in Verbindung bringt,

e. eine Spülkammer (10), wobei die Spülkammer (10) mit der Ausgleichskammer (4) mittels einer ersten Öffnung (20) und mit der Verteilebene (3) oder Sammelebene (3a) mittels einer zweiten Öffnung (21) in Verbindung steht,

wobei die Vorrichtung dadurch charakterisiert ist, dass eine Versorgungsleitung (9) mit einem im wesentlichen reinen oder im wesentlichen nicht verschmutzten zweiten Fluid unmittelbar mit der Spülkammer an einem Punkt so verbunden ist, dass ein Teil des zweiten Fluids von der Spülkammer (10) zu der Druckausgleichskammer über die erste Ausgangsöffnung (20) zirkulieren kann und dass der verbleibende Teil von der Spülkammer (10) zu der Separationskammer oder Reaktionskammer über die zweite Öffnung (21) zirkulieren kann und dass die besagte Druckausgleichskammer (4, 4a) mit einem Mittel (14) zur Evakuierung des Fluids, das sie enthält, verbunden ist.

**2.** Behälter nach dem Anspruch 1, in welcher die Spülkammer röhrenförmig ist.

**3.** Behälter nach dem Anspruch 1 oder 2, in welcher die Spülkammer im Inneren der Ausgleichskammer (4, 4a) gelegen und durch die Verteilebene (3) oder Sammelebene (3a) begrenzt ist und in welcher die Versorgungsleitung (9) des zweiten Fluids mit der Spülkammer (10) an einem Punkt verbunden ist, dar einem 30 - 90% des Gesamtvolumens der besagten Kammer darstellenden Volumen der Spülkammer, von der ersten Stirnfläche der Ebene bestimmt, entspricht.

**4.** Behälter nach dem Anspruch 3, in welcher der besagte Verbindungspunkt einem 50 - 80 % des Gesamtvolumens der Spülkammer darstellenden Volumen entspricht.

**5.** Behälter nach einem der Ansprüche 1 - 4, in welcher die Spülkammer die Ausgangsöffnung (20) auf der Seite der Druckausgleichskammer, in unmittelbarer Nachbarschaft von der ersten Stirnfläche der Ebene, hat.

**6.** Behälter nach einem der Ansprüche 2 - 5, in welcher das Mittel zur Evakuierung (14) des Fluids der Druckausgleichskammer in unmittelbarer Nachbarschaft von der besagten Stimfläche der Ebene gelegen ist.

**7.** Behälter nach einem der Ansprüche 5 - 6, in welcher die Ausgangsöffnung (20) der Spülkammer, auf Seiten der Druckausgleichskammer, im wesentlichen diametral entgegengesetzt dem Mittel zur Evakuierung (14) des in der Druckausgleichskammer enthaltenen Fluids ist

**8.** Behälter nach einem der Ansprüche 1 - 7, in welcher die Kammer zur chromatographischen Separation ein blindes Fließbett ist

**9.** Verfahren zum Druckausgleich und Spülen in einer Kammer zur chromatographischen Separation oder in einer Reaktionskammer, umfassend an einem Ende einen Druckausgleichsbereich (4) und einen Chromatographie- oder Reaktions- (5) Bereich, in welchem man ein erstes Fluid über eine Verteilebene (3) oder Sammelebene (3a), die den Druckausgleichsbereich und den Chromatographie- oder Reaktionsbereich von einander trennen, zirkulieren lässt, wobei das Verfahren dadurch charakterisiert ist, dass man ein zweites aus der durch ein Lösungsmittel, ein zu untersuchendes Produkt und deren Gemisch in einem Spülbereich (10), der den besagten Chromatographie- oder Reaktionsbereich und den Druckausgleichsbereich miteinander verbindet, gebildeten Gruppe ausgewähltes Fluid derartig zirkulieren lässt, dass ein Teil des zweiten Fluids von der besagten Spülkammer zu dem Druckausgleichsbereich (4) zirkuliert und dass der verbleibende Teil des zweiten Fluids von der Spülkammer zu dem Chromatographie- (5) oder Reaktionsbereich zirkuliert und dass man eine der den Spülbereich verlassenden Fluidmenge entsprechende Menge eines aus der Druckausgleichskammer stammenden Fluids evakuieren lässt.

10. Verfahren nach dem Anspruch 9, in welchem die direkte Verbindung auf dem Spülbereich derart positioniert ist, dass man ein Sicherheitsvolumen zwischen dem Verbindungspunkt und der Verteil- oder Sammelebene bestimmt, welches 30 - 90 % des Volumens des Spülbereichs darstellt und ausreichend groß ist, dass kein Transfer von Fluid aus dem Chromatographie- oder Reaktionsbereich zur Druckausgleichszone bei Druckschlägen auftritt.

11. Verfahren nach Anspruch 9 oder 10, in welchem man ein zweites Fluid mit einem Durchfluss $d_1$ mindestens gleich 0,3% des zu dem ersten Fluid gehörenden Durchflusses einleitet.

12. Verfahren nach einem der Ansprüche 9 - 11, in welchem man einen Durchfluss d1 des zweiten Fluids festsetzt, der unmittelbar in den Spülbereich eindringt. Man regelt einen Durchfluss $d_2$ des den Druckausgleichsbereich verlassenden Fluids derart, dass der Durchfluss $d_3$ des in den Chromatographie- oder Reaktionsbereich (5) strömenden zweiten Fluids über die Öffnung (21) mindestens gleich 5 %, vorteilhaft mindestens gleich 10 % des Durchflusses $d_1$ und vorzugsweise zwischen 40 und 60 % des Durchflusses $d_1$ liegt.

13. Verwendung der Behälter nach einem der Ansprüche 1 - 8 für die chromatographische Separation eines Kohlenwasserstoffgemisches, welches Xylole, bevorzugt im blinden Fließbett, umfasst.

14. Verwendung der Behälter nach einem der Ansprüche 1 - 8 zur chromatographischen Separation eines Gemisches aus aliphatischen Kohlenwasserstoffen.

**Claims**

1. A vessel comprising an apparatus for equalising pressure and flush, said vessel (1) having at least one concave extremity, the vessel comprising:

   - a chromatographic separation chamber (5) or a reaction chamber,
   - at least one distribution plate (3) or collection plate (3a) located in the vessel,
       that is in communication with said separation chamber (5) or reaction chamber,
   - a pressure equalisation chamber (4, 4a),

said equalisation chamber (4, 4a) being defined at least in part by the extremity of said vessel and by a first face of the plate, and said separation chamber (5) or reaction chamber being at least partially defined by a second face of said distribution or collection plate,
   - a first conduit (6) which places a first fluid in communication with the distribution or collection plate,
   - a flush chamber (10),

said flush chamber (10) being in communication with the equalisation chamber (4) by a first opening (20), and with the distribution plate (3) or collection plate (3a) by a second opening (21),
the apparatus being **characterized in that** a line (9) for supplying a second fluid which is substantially pure or substantially non contaminated is directly connected to the flush chamber at a point such that a portion of said second fluid can circulate from the flush chamber (10) towards the pressure equalisation chamber via the first outlet opening (20) and the remaining portion can circulate from the flush chamber (10) towards the separation or reaction chamber via the second opening (21), and **in that** said pressure equalisation chamber (4, 4a) is connected to a means (14) for evacuating the fluid it contains.

2. A vessel according to claim 1, in which the flush chamber is tubular.

3. A vessel according to claim 1 or claim 2, in which the flush chamber is located inside the equalisation chamber (4, 4a) and delimited by the distribution plate (3) or collection plate (3a), and in which the line (9) supplying the second fluid is connected to the flush chamber (10) at a point corresponding to a volume inside the flush chamber which represents 30% to 90% of the total volume of the flush chamber, this volume being determined from the first face of the plate.

4. A vessel according to claim 3, in which said connection point corresponds to a volume representing 50% to 80% of the total volume of the flush chamber.

5. A vessel according to any one of claims 1 to 4, in which the flush chamber comprises an outlet opening (20) on the pressure equalisation side, in the immediate vicinity of the first face of the plate.

6. A vessel according to any one of claims 2 to 5, in which the means (14) for evacuating fluid from the pressure equalisation chamber is located in the immediate vicinity of said first face of the plate.

**7.** A vessel according to claim 5 or claim 6, in which the outlet opening (20) from the flush chamber, on the pressure equalisation side, issubstantially diametrically opposite the means (14) for evacuating fluid from the pressure equalisation chamber.

**8.** A vessel according to any one of claims 1 to 7, in which the chromatographic separation chamber is a simulated moving bed.

**9.** A process for equalising pressure and flushing in a chromatographic separation chamber or in a reaction chamber comprising, at one extremity, a pressure equalisation zone (4), and a chromatographic or reaction zone (5) in which a first fluid is circulated via a distribution plate (3) or collection plate (3a) separating the pressure equalisation zone from the chromatographic or reaction zone, the process being **characterized in that** a second fluid selected from the group formed by a solvent, a desired product and a mixture thereof, is circulated in a flush zone (10) which places said chromatographic or reaction zone in communication with the pressure equalisation zone, by introducing said second fluid into the flush zone via a direct connection, such that a portion of the second fluid circulates from said flush chamber towards the pressure equalisation chamber (4) and the remaining portion of the second fluid circulates from the flush chamber towards the chromatographic or reaction zone (5), and **in that** a quantity of fluid originating from the pressure equalisation chamber is evacuated, which quantity corresponds to the quantity of fluid leaving the flush zone.

**10.** A process according to claim 9 in which the direct connection to the flush zone is positioned so as to determine a guard volume between the connecting point and the distribution or collection plate, representing 30% to 90% of the volume of the flush zone and sufficiently large for there to be no fluid transfer from the chromatographic or reaction zone to the pressure equalisation zone during sudden variations in pressure.

**11.** A process according to claim 9 or claim 10, in which the second fluid is introduced at a flow rate $d_1$ of at most 0.2% of the corresponding flow rate of the first fluid.

**12.** A process according to any one of claims 9 to 11, in which a flow rate $d_1$ of the second fluid penetrating directly into the flush zone is fixed, and a flow rate $d_2$ of fluid leaving the pressure equalisation zone is controlled so that the flow rate $d_3$ of the second fluid via the opening (21) flowing into the chromatographic or reaction zone (5) is at least 5% of flow rate $d_1$, advantageously at least 10%, and preferably in the range 40% to 60% of flow rate $d_1$.

**13.** Use of a vessel according to any one of claims 1 to 8 for chromatographic separation of a mixture of hydrocarbons comprising xylenes, preferably in a simulated moving bed.

**14.** Use of a vessel according to any one of claims 1 to 8 for the chromatographic separation of a mixture of aliphatic hydrocarbons.

**FIG.1**

**FIG.2**